# EUROPÄISCHE PATENTSCHRIFT

(11) **EP 0 934 922 B1**
(45) Veröffentlichungstag und Bekanntmachung des Hinweises auf die Patenterteilung: **07.04.2004**
(21) Anmeldenummer: 98124266.2
(22) Anmeldetag: 18.12.1998
(51) Int. Cl.: C07C 209/78, C07C 211/50

(54) **Verfahren zur Herstellung von Methylendi(phenylamin)**
Process for the preparation of methylenedi(phenylamine)
Procédé pour la préparation de di(phénylamine) de méthylène

(30) Priorität: 07.02.1998 DE 19804916
(43) Veröffentlichungstag der Anmeldung: 11.08.1999
(73) Patentinhaber: BASF AKTIENGESELLSCHAFT, 67056 Ludwigshafen (DE)
(72) Erfinder: Ströfer, Eckhard, Dr., 68163 Mannheim (DE); Caesar, Friedrich, Dr., 67069 Ludwigshafen (DE); Penzel, Ulrich, Dr., 01945 Tettau (DE); Schorr, Volker, 01968 Senftenberg (DE); Gmerek, Karl-Heint, 01968 Senftenberg (DE); Hasse, Hans, Dr., 67661 Kaiserslautern (DE)

(56) Entgegenhaltungen:
- EP-A- 0 451 442
- DD-A- 295 628
- GB-A- 1 450 632

## Beschreibung

Die Erfindung betrifft Verfahren zur Herstellung von Methylendi(phenylamin) durch Umsetzung von Anilin mit Formaldehyd in Gegenwart saurer Katalysatoren.

Die Herstellung von Methylendi(phenylamin), im Folgenden auch als MDA bezeichnet, ist allgemein bekannt und erfolgt üblicherweise durch kontinuierliche oder diskontinuierliche Umsetzung von Anilin mit Formaldehyd in Gegenwart von sauren Katalysatoren. Bei dieser Umsetzung, deren Hauptprodukt das 4,4'-MDA ist, wird in geringem Ausmaß das unerwünschte Nebenprodukt N-Methyl-MDA gebildet. Dieses Nebenprodukt wirkt sich insbesondere bei der anschließenden Umsetzung des MDAs mit Phosgen zur Herstellung von Methylendi(phenylisocyanat), auch als MDI bezeichnet, negativ aus, da das N-Methyl-MDA die Vorläuferverbindung für chlorierte Nebenprodukte im MDI darstellt und möglichst geringe Gehalte an Chlor im MDI angestrebt werden.

Zur Verringerung von N-Methyl-MDA als Nebenprodukt bei der Herstellung von MDA sind verschiedene Verfahren bekannt.

GB-A 1 450 632 offenbart Verfahren zur Herstellung von MDA aus Anilin und Formaldehyd in Gegenwart von wässriger HCl.

So beschreibt US 5 286 760 für eine kontinuierliche MDA Herstellung eine Teilneutralisierung des Reaktionsgemisches zwischen der Kondensationsstufe von zwei Molekülen Anilin und einem Molekül Formaldehyd und der anschließenden Umlagerung der intermediär gebildeten Aminobenzylamine, abgekürzt ABA, zum MDA.

EP-A 451 442 und DD-A 238 042 offenbaren für ein kontinuierliches Verfahren die Zugabe von Formaldehyd über mehrere Verfahrensstufen.

Auch für diskontinuierliche Verfahren sind Verfahren zur Verringerung des Nebenproduktes bekannt. DD-A 295 628 beschreibt die Zugabe des Formaldehydes in zwei Schritten während der Kondensationsstufe, wobei in der ersten Zugabe die Hauptmenge des Formaldehydes bei niederer Temperatur erfolgt und die zweite Zugabe des restlichen Formaldehydes bei gleicher oder höherer Temperatur erfolgt.

Nachteilig bei diesen Verfahren ist die nicht ausreichende Absenkung des Gehaltes an N-Methyl-MDA im Produktgemisch, so daß weiterhin ein Bedarf zur Verbesserung existiert.

Der vorliegenden Erfindung lag deshalb die Aufgabe zugrunde, ein Verfahren zur Herstellung von Methylendi(phenylamin) zu entwikkeln, durch das der Gehalt an N-Methyl-MDA als unerwünschtes Nebenprodukt minimiert wird.

Diese Aufgabe konnte erfindungsgemäß dadurch gelöst werden, daß man ein Gemisch (a) einsetzt, das (b) Poly(oxymethylen)glykole und gegebenenfalls (c) monomeren Formaldehyd und/oder Methylenglykol enthält, wobei das Molverhältnis von (b):(c) größer 1:0,6, bevorzugt 1:0,4 bis 1:0 beträgt und man das Gemisch (a) aus einer wässrigen Formaldehydlösung in einer Destillationseinrichtung oder Reaktionskolonne herstellt.

Monomerer Formaldehyd und die höheren Homologen des Formaldehydes, wie z.B. die Poly(oxymethylen)glykole, stehen in wässrigen Lösung miteinander in einem Gleichgewicht. Dies bedeutet, daß die höheren Homologen des Formaldehydes nach Zugabe zu dem Anilin und sauren Katalysator enthaltenden Reaktionsgemisch zum Formaldehyd abgebaut werden. Eine direkte Zugabe von monomeren Formaldehyd oder Methylenglykol zum Anilin wirkt sich nachteilig auf die Umsetzung zum MDA aus, da auch bei sehr intensiver Durchmischung nicht ausgeschlossen werden kann, daß lokal sehr hohe Konzentrationen an Formaldehyd oder Methylenglykol im Verhältnis zum Anilin vorliegen. Diese ungünstigen lokalen Konzentrationsverhältnisse fördern die Bildung des unerwünschten Nebenproduktes N-Methyl-MDA, da nach einer ersten Kondensation eines Moleküls Anilin mit einem Molekül Formaldehyd das Produkt dieser Kondensation entweder mit einem weiteren Molekül Anilin in Richtung des gewünschten ABAs oder MDAs reagieren kann, oder mit einem weiteren Molekül Formaldehyd in Richtung der unerwünschten N-Methyl-Verbindung. Durch die Zugabe des Formaldehydes in Form der höheren Homologe zum Anilin ergibt sich somit der wesentliche Vorteil, daß sehr hohe lokale Konzentrationen an Formaldehyd bei der Durchmischung mit dem Anilin ausgeschlossen werden können. Das Formaldehyd wird durch den Abbau der höheren Homologe in dem Reaktionsgemisch, enthaltend das Anilin für die Kondensation, mit dem Anilin bereitgestellt, wobei die höheren Homologe bevorzugt homogen in dem Reaktionsgemisch gelöst, dispergiert oder emulgiert vorliegen. Die Bildung des unerwünschtem Nebenproduktes N-Methyl-MDA wird damit unterdrückt.

Bevorzugt wird ein Gemisch eingesetzt, in dem das Molverhältnis von Poly(oxymethylen)glykolen mit beispielsweise 2 bis 9 Kohlenstoffatomen [Di(oxymethylen)glykol, Tri(oxymethylen)glykol, Tetra(oxymethylen)glykol, Penta(oxymethylen)glykol, Hexa(oxymethylen)glykol, Hepta(oxymethylen)glykol, Okta(oxymethylen)glykol und Nona(oxamethylen)glykol] als (b) zu monomeren Formaldehyd und Methylenglykol (c) > 1:0,6, bevorzugt 1:0,4 bis 1:0 beträgt. Das Gemisch (a), das die höheren Homologe des Formaldehydes enthält, wird aus einer üblichen Formaldehydlösung durch bekannte Verfahren zur Verschiebung des Gleichgewichtes auf die Seite der höheren Homologe hergestellt, d.h. aus einer wässrigen Formaldehydlösung in einer Destillationseinrichtung, einem Dünnschichtverdampfer oder einer Reaktionskolonne. Z.B. kann eine Formaldehydlösung bei einer Temperatur von 50 bis 150 °C beispielsweise in einer Reaktionskolonne entspannt werden, indem man den Druck, unter dem die Lösung steht, stark vermindert, wodurch die höheren Homologen des Formaldehydes in der Lösung verbleiben und monomeres aus Methylenglykol gebildetes Formaldehyd verdampft. Da das Gleichgewicht zwischen den höheren Homologen des Formaldehydes und dem monomeren Formaldehyd in der Lösung bei üblichen Temperaturen von 30 bis 70 °C innerhalb von 0,5 bis 15 Minuten wieder eingestellt ist, d.h. die erfindungsgemäße Anreicherung an höheren Homologen in der Lösung nicht mehr vorliegt, setzt man die Lösung mit dem erfindungsgemäß hohen Anteil an höheren Homologen des Formaldehydes bevorzugt innerhalb von 10 Minuten nach deren Herstellung in dem erfindungsgemäßen Verfahren zur Herstellung von MDA ein. Die Lösung kann als Gemisch (a) in dem erfindungsgemäßen Verfahren verwendet werden. Es ist weiterhin möglich, daß bei der Herstellung und Reinigung von Formaldehyd aus einer entsprechenden Destillationskolonne Fraktionen entnommen werden können, die den erfindungsgemäßen Anteil an höheren Homologen des Formaldehydes aufweisen. Das Gemisch (a) kann man in flüssiger und/oder gasförmiger Phase einsetzen.

Der erfindungsgemäße Einsatz des Gemisches (a) kann in allgemein bekannten Verfahren zur Herstellung von MDA erfolgen, beispielsweise kontinuierlichen, halbkontinuierlichen oder diskontinuierlichen Verfahren in üblichen Reaktoren oder Reaktorkombinationen, beispielsweise Rohrreaktoren, Rührkesseln mit oder ohne äußeren Umläufen, Schlaufenreaktoren, Rührkesselkaskaden oder Kombinationen obiger Reaktortypen. Diese Reaktoren oder Reaktorkombinationen können seriell verschaltet sein, aber auch reaktorübergreifend Rückführungen aufweisen. Das Gemisch (a) kann in dem erfindungsgemäßen Verfahren in flüssiger oder gasförmiger Form eingesetzt werden.

Zudem ist möglich, ein Gemisch (a) einzusetzen, das Paraformaldehyd als höheres Homologes des Formaldehydes enthält und das wie die beschriebenen Poly(oxymethylen)glykole in einem Gleichgewicht mit dem monomeren Formaldehyd steht. Das Paraformaldehyd kann als Feststoff in dem erfindungsgemäßen Verfahren eingesetzt werden.

Die Ausgangskomponenten (a) enthaltend (b) und (c), Anilin und saurer Katalysator können in üblichen Reinheitsgraden verwendet werden. Als saurer Katalysator können allgemein für diese Umsetzung bekannte Katalysatoren verwendet werden, bevorzugt wird HCl eingesetzt. Als Gemisch (a) können beispielsweise wässrige Lösungen mit einem Gehalt an (b) plus (c) von 5 bis 60 Gew.-%, bezogen auf das Gewicht des Gemisches (a), verwendet werden.

Das molare Verhältnis von Anilin zu saurem Katalysator in dem Reaktionsgemisch beträgt üblicherweise 1:0,6 bis 1:0,01, bevorzugt 1:0,3 bis 1:0,05.

Das molare Verhältnis von eingesetztem Formaldehyd, das bei vollständigem Abbau von dem in (a) enthaltenen (b) zu (c) in dem Gemisch (a) vorliegt, im Folgendem auch Gesamtformaldehydgehalt genannt, zu eingesetztem Anilin beträgt üblicherweise 1:1,7 bis 1:7,2, bevorzugt 1:1,9 bis 1:5,1.

Das Gemisch (a) wird bevorzugt durch eine Reaktionsmischpumpe oder eine Düse mit dem Reaktionsgemisch, dem Anilin oder Anilin/ Säuregemisch in Kontakt gebracht, d.h. in den Reaktor, in der die Umsetzung mit dem Anilin erfolgen soll, eingespeist. Um unerwünschte Parallelreaktionen zu Nebenprodukten zu vermeiden, erfolgt die Zugabe des Gemisches (a) bevorzugt derart, daß eine möglichst rasche und vollständige Durchmischung mit dem Reaktionsgemisch, das sich in der Apparatur befindet, stattfindet. Dies kann beispielsweise durch die Erzeugung einer turbulenten Strömung in der Mischkammer erreicht werden.

Die erfindungsgemäße Umsetzung von Anilin mit Formaldehyd in Gegenwart von sauren Katalysatoren wird üblicherweise bei Temperaturen von 20 bis 160°C, bevorzugt bei einer Temperatur von 20 bis 75°C durchgeführt. Die Temperatur hat unter anderem einen Einfluß auf die Isomerenverteilung des Methylendi(phenylamins) im Produkt. Falls bevorzugt die 2,2'- und/oder 2,4'-Methylendi(phenylamine) hergestellt werden sollen, kann eine hohe Temperatur vorteilhaft sein. Die Temperierung des Reaktionsgemisches kann mit allgemein üblichen Einrichtungen durchgeführt werden.

Die Umsetzung kann derart erfolgen, daß man kontinuierlich Anilin und/oder sauren Katalysator sowie Gemisch (a) in den Reaktor, in dem die Umsetzung erfolgen soll, einspeist und kontinuierlich Produkt aus dem Kreislauf entnimmt, so daß eine Volumenkonstanz in dem Reaktor erreicht wird. Dabei sollten die pro Zeiteinheit eingespeisten Mengen an Anilin, saurem Katalysator und Gesamtformaldehydgehalt bevorzugt in den eingangs beschriebenen molaren Verhältnissen erfolgen.

Es ist ebenso möglich, daß man in einem Reaktor Anilin und bevorzugt HCl als sauren Katalysator vorlegt, mischt, und diesem Reaktionsgemisch, bevorzugt durch eine Reaktionsmischpumpe oder Düse, Gemisch (a) zugibt. Die Zugabe von (a) kann derart erfolgen, daß konstante Volumina pro Zeiteinheit in das Reaktionsgemisch eingespeist werden, bis ein geeignetes molares Verhältnis von Anilin zu Gesamtformaldehydgehalt in dem Reaktionsgemisch vorliegt. Bevorzugt erfolgt die Zugabe derart, daß pro Minute 0,05 bis 2 % des ursprünglichen Volumens des Anilins in Apparatur als Volumen des Gemisches (a) in das Reaktionsgemisch geleitet werden. Anstatt der Einleitung des konstanten Volumens des Formaldehydes pro Zeiteinheit kann das Formaldehyd derart dem Reaktionsgemisch zudosiert werden, daß das Volumen des Gemisches (a), das pro Zeiteinheit zugegeben wird, gemäß einer mathematischen Funktion mit dem Fortschritt der Zugabe sinkt. Bevorzugt ist eine lineare oder exponentiell fallende Zugabefunktion.

Des Weiteren kann man das Gemisch (a) gepulst in das Reaktionsgemisch einführen, wobei eine regelmäßige oder unregelmäßige Pulsfrequenz und Zugabemenge gewählt werden kann. Der insgesamt einzuleitende Gesamtformaldehydgehalt sollte bevorzugt den eingangs beschriebenen molaren Verhältnissen in Bezug auf die Anilinmenge entsprechen. Bei dieser diskontinuierlichen Verfahrensweise wird das Reaktionsgemisch nach gewünschtem Umsatz aus der Apparatur entleert und gegebenenfalls weiter aufgearbeitet.

Die erfindungsgemäße Umsetzung kann beispielsweise in einer Apparatur durchgeführt werden, die
1) Zuleitungen für Anilin und sauren Katalysator,
2) Zuleitung für das Gemisch (a),
3) mindestens eine Reaktionsmischpumpe oder Düse, durch die das Gemisch (a) in die Apparatur eingespeist wird,
4) mindestens einen Reaktor mit oder ohne
5) Einrichtungen zur Durchmischung des Reaktionsgemisches,
6) ein Rohrsystem, das ausgehend vom Reaktor einen Kreislauf des Reaktionsgemisches ermöglicht,
7) eine Einrichtung zur Temperierung des Reaktionsgemisches und
8) gegebenenfalls eine Pumpe, die das Reaktionsgemisch in (6) in einem Kreislauf bewegt,
9) mindestens einen Anschluß zur Entnahme des Reaktionsgemisches aufweist.

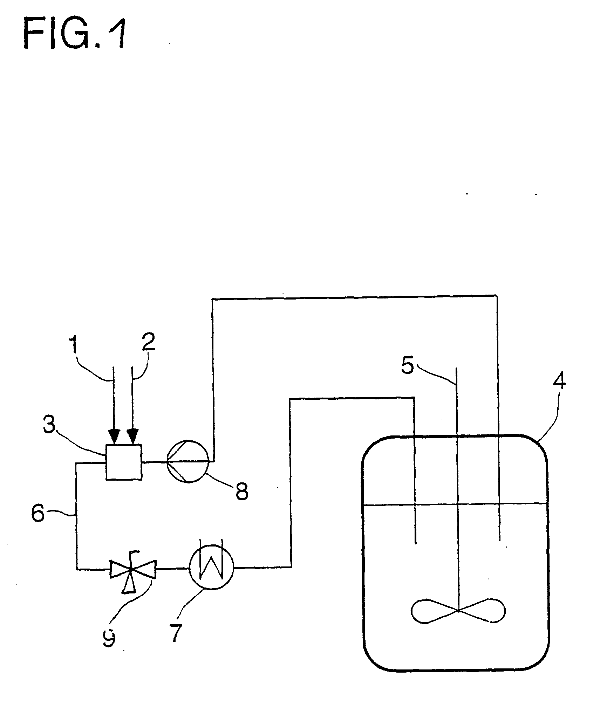

Eine solche Apparatur ist beispielhaft in Figur 1 dargestellt, wobei anzumerken ist, daß die Zugabe von Anilin und saurem Katalysator sowohl gemeinsam, wie in Figur 1 dargestellt, als auch getrennt, an weitgehend beliebiger Stelle der Apparatur, beispielsweise durch Zugabe in den Reaktor (4) oder durch Anschlüsse an der Reaktionsmischpumpe oder Düse (3), erfolgen kann. Auch die Anschlüsse 7, 8 und 9 können weitgehend beliebig, beispielsweise im Falle des Anschlusses 9 auch am Reaktor 4 angeordnet sein. Eine Mischeinrichtung (5) kann, wie in Figur 1 dargestellt, als Rührer in dem Reaktor (4) angeordnet sein. Die Durchmischung des Reaktionsgemisches kann durch die Pumpe (8) wahrgenommen werden.

Das Volumen des Reaktors (4) kann je nach gewünschtem Umsatz variabel gewählt werden. Auch die Durchmesser von Reaktor und Rohrsystem, die variabel gewählt werden können, und die Länge des Rohrsystems (6) können je nach Ansatzgröße weitgehend frei gewählt werden, wobei das Reaktionsgemisch bei der gewählten Strömungsgeschwindigkeit und dem Durchmesser der Rohre in dem Rohrsystem bevorzugt in turbulenter Strömung fließt. Für die Komponenten (1) bis (9) können, wie für die Komponenten (3) und (7) bereits dargestellt, übliche Einrichtungen verwendet werden. Eine zur Durchführung des erfindungsgemäßen Verfahrens geeignete Apparatur kann aus für diesen Zweck üblichen Materialien, beispielsweise Stahl üblicher Zusammensetzung und Qualität, bestehen.

Die Verweilzeit- und verteilung des Reaktionsgemisches in der Apparatur ist abhängig von den Einrichtungen der Apparatur und der gewählten Temperatur.

Nach der erfindungsgemäßen Umsetzung von Formaldehydes mit Anilin kann das Reaktionsgemisch gegebenenfalls in weitere Reaktoren, beispielsweise Rohrreaktoren, Rührkesseln, Rührkesselkaskaden, Kombinationen aus Rührkesseln und Rohrreaktoren, geleitet werden, in denen gegebenenfalls die Umsetzung zum MDA vervollständigt werden kann.

Das Reaktionsgemisch kann nach der Umsetzung durch allgemein bekannte Verfahren aufgearbeitet werden, beispielsweise durch Neutralisation, Phasentrennung, Destillation und/oder chromatographische Trennmethoden.

Das Verfahrensprodukt, d.h. das Gemisch enthaltend Metyhlendi-(phenylamin), beispielsweise 2,2'-, 2,4', und/oder 4,4'-MDA, enthält bevorzugt weniger als 0,05 Gew.-% N-Methyl MDA und wird bevorzugt zur bekannten Synthese von MDI und polymerem MDI beispielsweise durch übliche Phosgenierung von Polyaminen eingesetzt. Die Phosgenierung kann beispielsweise in üblichen, bevorzugt inerten Lösungsmitteln, z.B. Mono- und/oder Dichlorbenzol, in üblichen Reaktoren, beispielsweise Rührkesseln, Rührkesselkaskaden und/oder Rohrreaktoren bei bekannten Temperaturen von z.B. 50 bis 150°C, bevorzugt 70 bis 120°C durchgeführt werden. Das durch die Phosgenierung hergestellte Roh-MDI kann durch übliche Verfahren, beispielsweise Destillation, gereinigt werden. Z.B. kann in einem ersten Vorgang das Lösungsmittel abdestilliert werden und anschließend in einer zweiten Destillation das gewünschte erfindungsgemäße 2,2'-, 2,4' und/oder 4,4'-MDI. Diese mit dem erfindungsgemäßen Methylendi(phenylamin) hergestellten Polyisocyanate weisen insbesondere den Vorteil auf, daß sie einen geringen Gehalt an hydrolisierbarem Chlor besitzen.

Das erfindungsgemäße Verfahren soll anhand der folgenden Beispiele näher dargestellt werden.

### Vergleichsbeispiel 1:

Die Umsetzung wurde in einer Apparatur durchgeführt, die aus einer Rührkesselkaskade mit drei Reaktoren, die Volumina von 700, 800 und 800 ml auswiesen, und einem mit Füllkörpern gepackten Rohr bestand. Die Reaktionstemperaturen in den Reaktoren wurden mittels externer Kühlung bzw. Heizung auf 50 (erster Rührkessel), 70 (zweiter Rührkessel), 80 (dritter Rührkessel) und 100°C (Rohrreaktor) eingestellt. Das mit Füllkörpern gepackte Rohr wies ein Gesamtvolumen von 5000 ml und einen inneren Rohrdurchmesser von 30 mm auf. Die Drehzahl der Rührer in den Reaktoren der Rührkesselkaskade betrug jeweils 500 U/min. In den ersten Reaktor wurde Anilin mit 1400 g/h zugegeben, welches zuvor mit 450 g/h 30 %iger wässriger Salzsäure vermischt worden war. An dem ersten Reaktor befand sich ein äußerer Umpumpkreis mit einem dynamischen Mischer, in den 190 g/h einer im Gleichgewicht befindliche 50 %ige Formaldehydlösung in Wasser mittels eine Pumpe eingegeben wurde. Das Molverhältnis von Poly(oxymethylen)glykolen zu monomeren Formaldehyd und Methylenglykol in dieser Formaldehydlösung betrug 1:0,7. Das Produktgemisch aus dem Rohrreaktor wurde Natronlauge neutralisiert. Anschließend wurde eine Phasentrennung bei einer Temperatur von 70 bis 80°C vorgenommen. Die organische Phase wurde abgetrennt, mit dem 1,5-fachen Volumen an warmen Wasser gewaschen. Aus dieser gereinigten Phase wurde überschüssiges Anilin unter vermindertem Druck abdestilliert und in den ersten Reaktor zurückgeführt. 24 h nach dem Anfahren der Anlage war die Reaktionsmischung in einem stationären Zustand und es wurden Proben der organischen Phase genommen. Der Gehalt an N-Methyl MDA in dem erhaltenen Produkt betrug 0,07 Gew.-%. Diese Polyamin wurde in einem üblichen Verfahren zur Herstellung von Isocyanaten in einem zweistufigen Prozeß mit Phosgen umgesetzt. Der Gehalt an hydrolisierbarem Chlor in diesem Polyisocyanat betrug 0,09 %.

### Beispiel 1:

Es wurden einem Verfahren entsprechend Vergleichsbeispiel MDA hergestellt, mit dem einzigen Unterschied, daß anstelle der im Gleichgewicht befindlichen wässrigen Formaldehydlösung ein Gemisch eingesetzt wurde, in dem Poly(oxymethylen)glykole gegenüber Formaldehyd und Methylenglykol angereichert waren. Das Molverhältnis von Poly(oxymethylen)glykolen zu monomeren Formaldehyd und Methylenglykolen in diesem Gemisch betrug 1:0,3. Dieses Gemisch wurde durch schnelles Verdampfen von Formaldehyd aus dem ursprünglich im Gleichgewicht befindlichen Gemisch in einer Reaktionskolonne gewonnen. 24 h nach dem Anfahren der Anlage war die Reaktionsmischung in einem stationären Zustand und es wurden Proben der organischen Phase genommen. Der Gehalt an N-Methyl MDA in dem erhaltenen Produkt betrug 0,04 Gew.-%. Diese Polyamin wurde in einem üblichen Verfahren zur Herstellung von Isocyanaten in einem zweistufigen Prozeß mit Phosgen umgesetzt. Der Gehalt an hydrolisierbarem Chlor in diesem Polyisocyanat betrug 0,05 %.

Die Aufgabe, ein Verfahren zu entwickeln, mit dem die unerwünschte Bildung von N-Methyl MDA vermindert wird, konnte somit durch das erfindungsgemäße Verfahren gelöst werden. Nicht nur der Gehalt an dem unerwünschten N-Methyl MDA konnte deutlich um 43 % verringert werden, auch der Gehalt an hydrolisierbarem Chlor in dem Polyisocyanat, das mit dem erfindungsgemäß hergestellten MDA produziert wurde, konnte drastisch um 44 % reduziert werden.

Sowohl das erfindungsgemäß hergestellte MDA als auch das mit diesem MDA produzierte Polyisocyanat wies somit wesentlich verbesserte Eigenschaften auf.

## Patentansprüche

1. Verfahren zur Herstellung von Metyhlendi(phenylamin) durch Umsetzung von Anilin mit Formaldehyd in Gegenwart saurer Katalysatoren, **dadurch gekennzeichnet, daß** man ein Gemisch (a) einsetzt, das (b) Poly(oxymethylen)glykole und gegebenenfalls (c) monomeren Formaldehyd und/oder Methylenglykol enthält, wobei das Molverhältnis von (b):(c) größer 1:0,6 beträgt und man das Gemisch (a) aus einer wässrigen Formaldehydlösung in einer Destillationseinrichtung oder Reaktionskolonne herstellt.

2. Verfahren nach Anspruch 1, **dadurch gekennzeichnet, daß** man das Gemisch (a) in flüssiger und/oder gasförmiger Phase einsetzt.

3. Verfahren nach Anspruch 1, **dadurch gekennzeichnet, daß** das Gemisch (a) Paraformaldehyd enthält.

4. Verfahren nach einem der Ansprüche 1 bis 3, **dadurch gekennzeichnet, daß** das molare Verhältnis von Formaldehyd, das bei vollständigem Abbau von dem in (a) enthaltenen (b) zu (c) in dem Gemisch (a) vorliegt, zu Anilin 1:1,7 bis 1:7,2 beträgt.

5. Verfahren nach einem der Ansprüche 1 bis 4, **dadurch gekennzeichnet, daß** das man das Gemisch (a) durch eine Reaktionsmischpumpe oder eine Düse mit dem Anilin in Kontakt bringt.

6. Verfahren nach einem der Ansprüche 1 bis 5, **dadurch gekennzeichnet, daß** das molare Verhältnis von Anilin zu saurem Katalysator 1:0,6 bis 1:0,01 beträgt.

7. Verfahren nach einem der Ansprüche 1 bis 6, **dadurch gekennzeichnet, daß** man die Umsetzung bei einer Temperatur von 20 bis 160 °C durchführt.

## Claims

1. A process for preparing methylenedianiline by reacting aniline with formaldehyde in the presence of acid catalysts, which comprises using a mixture (a) which comprises (b) polyl(oxymethylene glycol)s with or without (c) monomeric formaldehyde and/or methylene glycol, the molar ratio of (b): (c) being greater than 1:0.6, and the mixture (a) being prepared from an aqueous formaldehyde solution in a distillation device or reaction column.

2. A process as claimed in claim 1, wherein the mixture (a) is used in the liquid and/or gaseous phase.

3. A process as claimed in claim 1, wherein the mixture (a) comprises paraformaldehyde.

4. A process as claimed in any one of claims 1 to 3, wherein the molar ratio of formaldehyde, which in the case of complete breakdown of the (b) present in (a) to form (c) in the mixture, to aniline is from 1:1.7 to 1:7.2.

5. A process as claimed in any one of claims 1 to 4, wherein the mixture (a) is contacted with the aniline by means of a reaction mixing pump or a nozzle.

6. A process as claimed in any one of claims 1 to 5, wherein the molar ratio of aniline to acid catalyst is from 1:0.6 to 1:0.01.

7. A process as claimed in any one of claims 1 to 6, wherein the reaction is carried out at a temperature of from 20 to 160°C.

## Revendications

1. Procédé de préparation de méthylènedi(phénylamine) par réaction d'aniline avec du formaldéhyde en présence de catalyseurs acides, **caractérisé en ce qu'**on utilise un mélange (a) qui contient (b) des poly(oxyméthylène)glycols et le cas échéant (c) du formaldéhyde et/ou du méthylèneglycol monomère, le rapport molaire de (b) :(c) étant supérieur à 1 :0,6, et **en ce qu'**on produit le mélange (a) à partir d'une solution aqueuse de formaldéhyde dans un dispositif de distillation ou une colonne de réaction.

2. Procédé selon la revendication 1, **caractérisé en ce qu'**on utilise le mélange (a) en phase liquide et/ou gazeuse.

3. Procédé selon la revendication 1, **caractérisé en ce que** le mélange (a) contient du paraformaldéhyde.

4. Procédé selon l'une des revendications 1 à 3, **caractérisé en ce que** le rapport molaire du formaldéhyde qui est présent dans la dégradation complète du (b) contenu dans (a) en (c) dans le mélange (a), par rapport à l'aniline, s'élève à 1 :1,7 à 1 :7,2.

5. Procédé selon l'une des revendications 1 à 4, **caractérisé en ce qu'**on met en contact avec l'aniline le mélange (a) au moyen d'une pompe de mélange de réaction ou d'une buse.

6. Procédé selon l'une des revendications 1 à 5, **caractérisé en ce que** le rapport molaire de l'aniline au catalyseur acide s'élève à 1 :0,6 à 1 :0,01.

7. Procédé selon l'une des revendications 1 à 6, **caractérisé en ce qu'**on conduit la réaction à une température de 20 à 160°C.
